# EUROPEAN PATENT SPECIFICATION

(11) **EP 3 903 881 B1**
(45) Date of publication and mention of the grant of the patent: **30.04.2025**
(21) Application number: 19904677.2
(22) Date of filing: 24.12.2019
(51) Int. Cl.: A61P 3/10, A61K 9/00, A61K 35/39, C12N 5/0775, A61F 2/02

(54) **CELL TRANSPLANT KIT AND METHOD FOR MANUFACTURING THE KIT**
ZELLTRANSPLANTATIONSKIT UND VERFAHREN ZUR HERSTELLUNG DES KITS
KIT DE TRANSPLANTATION DE CELLULE ET PROCÉDÉ DE FABRICATION DU KIT

(30) Priority: 25.12.2018 JP 2018241499; 14.02.2019 JP 2019024592
(43) Date of publication of application: 03.11.2021
(73) Proprietor: FUJIFILM Corporation, Tokyo 106-8620 (JP)
(72) Inventor: KOGAWA Ryo, Ashigarakami-gun, Kanagawa 258-8577 (JP); NAKAMURA Kentaro, Ashigarakami-gun, Kanagawa 258-8577 (JP)
(74) Representative: Hoffmann Eitle
(86) International application number: PCT/JP2019/050494
(87) International publication number: WO 2020/138028

(56) References cited:
- EP-A1- 2 968 115
- EP-A1- 3 600 145
- EP-B1- 3 600 145
- WO-A1-2016/052504
- WO-A1-2018/172371
- WO-A2-2018/102077
- JP-A- 2013 503 014
- JP-A- 2016 204 302
- JP-A- 2017 524 768
- JP-A- H11 508 477
- US-B2- 10 835 486
- US-B2- 8 278 106
- SCHNEIDER, S. ET AL.: "Long-Term Graft Function of Adult Rat and Human Islets Encapsulated in Novel Alginate-Based Microcapsules After Transplantation in Immunocompetent Diabetic Mice", DIABETES, vol. 54, 2005, pages 687 - 693, XP055723249, ISSN: 0012-1797

## Description

### BACKGROUND OF THE INVENTION

### Field of the Invention

The present invention relates to a cell transplantation kit that is useful in the medical treatment of a specific disease by transplanting cells. The present invention further relates to a method for producing a cell transplantation kit.

### Description of the Related Art

A portal vein-mediated intrahepatic transplantation is currently the main method for pancreatic islet transplantation. However, a large number of pancreatic islets transplanted by the portal vein-mediated intrahepatic transplantation are killed by an immune reaction in a short time, and thus the transplantation is required to be repeated several times to obtain a sufficient transplantation effect. In addition, since the transplantation is carried out intravascularly, problems such as embolization and bleeding are unavoidable, and further, the isolation of the pancreatic islet is not easy. For solving the above problems, other transplantation sites have been tested. However, there has been a problem that the blood flow is insufficient in a transplantation site other than the portal vein-mediated transplantation site, particularly in the subcutaneous site, and the engraftment rate of the pancreatic islet is low as compared with that of other transplants. Therefore, it has been attempted to increase the engraftment rate of the pancreatic islet by preparing a transplantation site having abundant blood flow before transplanting the pancreatic islet.

Non-Patent Document 1 describes that in a case where a vascular bed is prepared, the engraftment properties of transplanted cells are improved. Patent Document 1 discloses a device for transplanting cells in a host body, where the device includes a porous scaffold that including at least one chamber having a proximal end portion and a distal end portion and includes at least one removable plug configured to be disposed inside of at least one chamber and/or at least inside a part of the porous scaffold, and the porous scaffolds include a polypropylene mesh. Patent Document 2 describes a neovascular bed forming tool which is embedded in a tissue, where the tool can create a cavity covered by capillaries, around the neovascular bed forming tool, when embedded. The tool described in Patent Document 2 is a tool that is used for creating a tissue having sufficient capillaries.

Another problem in pancreatic islet transplantation is the donor shortage. As a countermeasure against this problem, studies on pancreatic islet transplantation using a heterologous pancreatic islet have been actively being carried out. However, the realization of heterologous pancreatic islet transplantation requires the protection of pancreatic islet from the immune rejection to the heterologous transplantation. Studies on immunity isolating membranes using a hydrogel and the like are underway as a countermeasure against immune rejection in cell transplantation, which is not limited to pancreatic islet transplantation. Non-Patent Document 2 describes that cells are immunity-isolated with an alginate capsule.

### Prior Art Documents

### Patent Documents

Patent Document 1: JP2018-043013A
Patent Document 2: JP2000-178180A

### Non-Patent Documents

Non-Patent Document 1: Uematsu SS (2018) Transplantation. 102 (3): 387 to 395
Non-Patent Document 2: Stephan, S et al. (2005) Diabetes. 54 (3): 687 to 693

A kit for cell transplantation is known from the Document WO-A-2018/17237. This document discloses a cell transplantation kit comprising: a first sac-shaped structure consisting of a membrane having pores of an average pore diameter of 0.2 mm or less and a plurality of second sac-shaped structures having an average long diameter larger than the average pore diameter of the pores of the first sac-shaped structure wherein at least a part of the plurality of second sac-shaped structures contain cells.

### SUMMARY OF THE INVENTION

An object of the present invention is to provide a cell transplantation kit capable of exhibiting a high medical treatment effect by transplanted cells, a method for producing a cell transplantation kit.

As a result of diligent studies to solve the above problems, the inventors of the present invention have found that in a case where a sac-shaped structure having minute pores into which blood vessels and immune cells are difficult to enter is transplanted in advance into a living body to prepare a cell transplantation site in advance, and then microencapsulated cells are transplanted to the above cell transplantation site, heterologous cells can be engrafted and a medical treatment effect can be exhibited. The present invention has been completed based on the above findings.

The present invention relates to a cell transplantion kit as set forth in the appended claims.

### BRIEF DESCRIPTION OF THE DRAWINGS

Fig. 1 is a schematic view illustrating an operation in a case where a pancreatic islet is transplanted using one example of a cell transplantation kit according to the embodiment of the present invention.
Fig. 2 shows a state 4 weeks after transplanting a first sac-shaped structure.
Fig. 3 shows a state at the time of transplanting a pancreatic islet.
Fig. 4 is a graph showing a transition of blood glucose level in a case where a heterologous pancreatic islet has been transplanted using a first sac-shaped structure made of nylon.
Fig. 5 is a graph showing a transition of body weight in a case where a heterologous pancreatic islet has been transplanted using a first sac-shaped structure made of nylon.
Fig. 6 shows immunostaining images of tissue slices 4 weeks after transplanting a cell structure (+) group and a cell structure (-) group.
Fig. 7 shows HE staining images of tissue slices 4 weeks after transplanting a cell structure (+) group and a cell structure (-) group.
Fig. 8 shows HE staining images of tissue slices around microcapsules 4 weeks after transplanting the cell structure (+) group and the cell structure (-) group.

### DESCRIPTION OF THE PREFERRED EMBODIMENTS

Hereinafter, embodiments for carrying out the present invention will be described in detail. In the present specification, "to" denotes a range including numerical values described before and after "to" as a minimum value and a maximum value, respectively.

A cell transplantation kit according to the embodiment of the present invention includes a first sac-shaped structure consisting of a membrane having pores of an average pore diameter of 0.2 mm or less, and a plurality of second sac-shaped structures having an average long diameter larger than the average pore diameter of the pores of the first sac-shaped structure, where at least a part of the plurality of second sac-shaped structures contains cells. According to the configuration of the present invention, cells have high engraftment properties and the transplanted cells can be protected from immune rejection. Further, since a sac-shaped structure is used, it is easily removed in the abnormal state, the addition or exchange of cells are easy, and thus a long-term medical treatment effect can be obtained. According to the present invention, it is possible to realize a cell transplantation therapy at a transplantation site other than the site where portal vein administration is carried out, using heterologous donor cells.

### <First sac-shaped structure>

The first sac-shaped structure consists of a membrane having pores having an average pore diameter of 0.2 mm or less.

The first sac-shaped structure has pores having an average pore diameter of 0.2 mm or less, preferably has pores having an average pore diameter of 50 µm or less, more preferably has pores having an average pore diameter of 20 µm or less, still more preferably has pores having an average pore diameter of 1 µm or less, and particularly preferably has pores having an average pore diameter of 0.05 µm or less.

In a case where the average pore diameter of the pores is 0.2 mm or less, the invasion of connective tissue, blood vessels, and immune cells into the first sac-shaped structure is not promoted.

As a method for measuring the average pore diameter of the first sac-shaped structure, 50 or more pores are continuously selected, the pore diameter of each of the selected pores is measured, and the average value is calculated as the average pore diameter. In addition, the pore diameter can also be measured by ASTM F316-80.

The opening ratio of the first sac-shaped structure is not particularly limited; however, it is preferably 25% to 85% and more preferably 40% to 75%.

The opening ratio indicates the ratio of the area occupied by the pores on the entire surface of the sac-shaped structure.

The opening ratio can be measured by calculating the ratio between the area of the opening part and the area of the membrane material in the image of the surface or cross-sectional view of the membrane taken vertically from the thickness direction of the membrane using an optical microscope or a scanning electron microscope (SEM).

The pore diameter of the first sac-shaped structure is preferably more uniform. However, in a case where the first sac-shaped structure has a pore diameter distribution in the thickness direction, the comparison of the pore diameters of the membrane in the thickness direction shall be carried out by dividing a scanning electron microscope (SEM) photomicrograph of the membrane cross section in the thickness direction of the membrane. The number of divisions can be appropriately selected depending on the thickness of the membrane. The number of divisions is at least 5, and the size of the division width means the size of the width of the membrane in the thickness direction but does not mean the size of the width on the photomicrograph. In the comparison of the pore diameters of the membrane in the thickness direction, the pore diameter is compared as the average pore diameter of each of the sections. The average pore diameter of each of the sections may be, for example, an average value of 50 pores in each of the sections of the membrane cross-sectional view.

The first sac-shaped structure is used for immunity isolation. Immunity isolation is a method for preventing immune rejection. In general, the immunity isolation is one of the methods for preventing the recipient's immune rejection at the time of transplantation. Here, the immune rejection is the recipient's rejection of the cells that are transplanted to the recipient. The immunity isolation isolates cells from the recipient's immune rejection. Examples of the immune rejection include the immune rejection due to a cell-mediated immune response and the immune rejection due to a humoral immune response.

The first sac-shaped structure is preferably a selectively permeable membrane that allows nutrients such as oxygen, water, and glucose to permeate and blocks the permeation of immune cells and the like involved in immune rejection. Examples of the immune cells include macrophages, dendritic cells, neutrophils, eosinophils, basophils, natural killer cells, various T cells, B cells, and other lymphocytes.

The first sac-shaped structure preferably blocks the permeation of high-molecular weight proteins such as an immunoglobulin (IgM or IgG) and complement and preferably allow physiologically active substances having a relatively low-molecular weight such as insulin to permeate, depending on the application.

The selective permeability of the first sac-shaped structure may be adjusted depending on the application. The first sac-shaped structure may be, for example, a selectively permeable membrane that blocks substances having a molecular weight of 500 kDa or more, 100 kDa or more, 80 kDa or more, or 50 kDa or more. For example, the first sac-shaped structure can preferably block the permeation of IgG (molecular weight: about 160 kDa), which is the smallest among the antibodies. In addition, the first sac-shaped structure may be a selectively permeable membrane that blocks substances having a diameter of 500 nm or more, 100 nm or more, 50 nm or more, or 10 nm or more, in terms of the size of the sphere.

The material of the first sac-shaped structure is suitably a material having high biocompatibility, and a material that does not induce a further inflammatory response is preferable. As the material of the first sac-shaped structure, nylon, polysulfone, polytetrafluoroethylene (PTFE), polyurethane, polyester, vinyl chloride, polycarbonate, acrylic, stainless steel, titanium, silicone, or 2-methacryloyloxyethyl phosphorylcholine (MPC) is preferable, nylon or polysulfone is more preferable.

The inside of the first sac-shaped structure may contain a cytokine or a substance having an anti-inflammatory action.

The inside of the first sac-shaped structure is provided with a cell structure.

The thickness of the membrane constituting the first sac-shaped structure is not particularly limited; however, it is preferably 10 µm to 500 µm. The lower limit of the thickness of the membrane constituting the first sac-shaped structure may be 20 µm or more, or 30 µm or more. The upper limit of the thickness of the membrane constituting the first sac-shaped structure may be 400 µm or less, 300 µm or less, 250 µm or less, 200 µm or less, or 100 µm or less.

The surface of the first sac-shaped structure may be coated with an extracellular matrix, a cell adhesive substance such as collagen, laminin, or vitronectin. The surface of the first sac-shaped structure may be coated with a cell non-adhesive coating agent.

The shape of the first sac-shaped structure may be a rectangular shape or an elliptical shape.

The first sac-shaped structure may be composed of a porous membrane.

The porous membrane refers to a membrane having a plurality of pores. The pores can be confirmed, for example, in a scanning electron microscope (SEM) image or a transmission electron microscope (TEM) image of the membrane cross section.

Preferably, the porous membrane has a layered dense portion having a minimum pore diameter in the inside thereof, and the pore diameter continuously increases from this dense portion toward at least one surface of the porous membrane in the thickness direction. The pore diameter shall be determined by the average pore diameter of the sections which will be described later.

In a case where a porous membrane has a pore diameter distribution, the life of the first sac-shaped structure can be improved. This is because the effect of carrying out multi-step filtration using membranes having substantially different pore diameters can be obtained, and the deterioration of the membranes can be prevented.

The pore diameter may be measured from a photomicrograph of a membrane cross section, obtained with an electron microscope. The porous membrane is cut with a microtome or the like to obtain thin membrane slices, with which the cross section can be observed, and a photomicrograph of the cross section of the porous membrane can be obtained.

The comparison of the pore diameters of the membrane in the thickness direction shall be carried out by dividing the SEM photomicrograph of the membrane cross section in the thickness direction of the membrane. The number of divisions can be appropriately selected depending on the thickness of the membrane. The number of divisions is at least 5, and for example, in a case of a membrane having a thickness of 200 µm, the membrane is divided to 20 divisions from the surface X which will be described later. The size of the division width means the size of the width of the membrane in the thickness direction but does not mean the size of the width on the photomicrograph. In the comparison of the pore diameters of the membrane in the thickness direction, the pore diameter is compared as the average pore diameter of each of the sections. The average pore diameter of each of the sections may be, for example, an average value of 50 pores in each of the sections of the membrane cross-sectional view. In this case, the cross-sectional view of the membrane may be obtained, for example, at a width of 80 µm (at a distance of 80 µm in a direction parallel to the surface). At this time, in a case of a section in which pores are large and thus 50 pores cannot be measured, pores that can be measured as many as possible in that section may be measured. Further, at this time, in a case where a pore is too large and does not fit in the section, the size of the pore is measured over the other section.

The layered dense portion having the minimum pore diameter refers to a layered portion of the porous membrane corresponding to the section having the smallest average pore diameter among the above-described membrane cross-section sections. The dense portion may consist of a portion corresponding to one section or may be consists of a portion corresponding to a plurality of sections such as two or three sections, which has an average pore diameter within 1.1 times that of the section having the smallest average pore diameter. The thickness of the dense portion may be 0.5 µm to 50 µm and preferably 0.5 µm to 30 µm.

In the present specification, the average pore diameter of the dense portion is defined as the minimum pore diameter of the porous membrane. The minimum pore diameter of the porous membrane is preferably 0.02 µm to 1.5 µm and more preferably 0.02 µm to 1.3 µm. This is because a porous membrane having such a minimum pore diameter can block at least ordinary cell permeation. Here, it is assumed that the average pore diameter of the dense portion is measured by ASTM F316-80.

The porous membrane has a dense portion in the inside thereof. The inside means space not being in contact with the surface of the membrane, and "having a dense portion in the inside thereof" means that the dense portion is not the section closest to any surface of the membrane. In the immunity isolating membrane, a porous membrane having a structure having a dense portion in the inside thereof is used, and thus the permeability of the substance that is intended to permeate is not easily reduced as compared with a case where a porous membrane having the same dense portion in contact with the surface thereof is used. It is not bound by any theory; however, it is presumed to be because the internal presence of the dense portion makes it difficult for protein adsorption to occur.

It is preferable that the dense portion is disposed toward any one of the surface sides rather than the central part of the porous membrane in the thickness direction. Specifically, it is preferable that the dense portion is disposed within two-fifths of the porous membrane from the surface of either one of the porous membranes in the thickness direction, more preferably within one-third, and still more preferably within one-fourth. This distance may be determined in the above-described cross-sectional photomicrograph of the membrane. In the present specification, the surface of the porous membrane closer to the dense portion is referred to as a "surface X".

In the porous membrane, the pore diameter continuously increases from the dense portion toward at least one surface in the thickness direction. In the porous membrane, the pore diameter may be continuously increased from the dense portion toward the surface X in the thickness direction, the pore diameter may be continuously increased from the dense portion toward the surface opposite to the surface X in the thickness direction, and the pore diameter may be continuously increased from the dense portion to any surface of the porous membrane in the thickness direction. Among the above, it is preferable that the pore diameter is continuously increased from at least the dense portion toward the surface opposite to the surface X in the thickness direction, and it is more preferable that the pore diameter is continuously increased from the dense portion to any surface of the porous membrane in the thickness direction. The description of "the pore diameter is continuously increased in the thickness direction" means that the pore diameter is increased such that the difference in the average pore diameter between adjacent sections in the thickness direction is 50% or less of the difference between the maximum average pore diameter (the maximum pore diameter) and the minimum average pore diameter (the minimum pore diameter), preferably 40% or less, and more preferably 30% or less. The description of "continuously increased" basically means that there is no decrease and the increase is uniform; however, the portion where decrease occurs may unintendedly exist. For example, in a case where two sections are each combined from the surface and in a case where the average value of the pore diameter of the combination is increased uniformly (decreased uniformly in a case of the direction from the surface to the dense portion), it can be determined that "the pore diameter is continuously increased from the dense portion to the surface of the membrane in the thickness direction".

The structure of the porous membrane in which the pore diameter is continuously increased in the thickness direction can be realized by, for example, a production method which will be described later.

In the first sac-shaped structure, the surface X of the porous membrane is disposed on the inner side. That is, it is preferable that the porous membrane is disposed so that the dense portion of the porous membrane is closer to the inside of the first sac-shaped structure. In a case where the surface X is disposed on the inner side of the first sac-shaped structure, the permeability of the physiologically active substance can be further increased.

The porous membrane generally contains a polymer. Basically the porous membrane is preferably composed of a polymer.

The polymer forming the porous membrane preferably has biocompatibility. Here, the "biocompatibility" includes the meaning of non-toxicity and non-allergenicity but does not include the property that a polymer is encapsulated in the living body.

The polymer preferably has a number-average molecular weight (Mn) of 1,000 to 10,000,000 and more preferably 5,000 to 1,000,000.

Examples of the polymer include a thermoplastic polymer and a thermosetting polymer. Specific examples of the polymer include polysulfone, cellulose acylate, nitrocellulose, sulfonated polysulfone, polyethersulfone, polyacrylonitrile, a styrene-acrylonitrile copolymer, a styrene-butadiene copolymer, a saponified product of an ethylene-vinyl acetate copolymer, polyvinyl alcohol, polycarbonate, an organosiloxane-polycarbonate copolymer, polyester carbonate, organopolysiloxane, polyphenylene oxide, polyamide, polyimide, polyamideimide, polybenzimidazole, an ethylene vinyl alcohol copolymer, and polytetrafluoroethylene (PTFE). These may be homopolymers, copolymers, or polymer blends, polymer alloys, from the viewpoints of solubility, optical properties, electrical properties, strength, elasticity, and the like.

Among the above, polysulfone or cellulose acylate is preferable, and polysulfone is more preferable.

The porous membrane may contain a component other than the polymer as an additive.

Examples of the additives include metal salts of inorganic acids such as common salt, lithium chloride, sodium nitrate, potassium nitrate, sodium sulfate, and zinc chloride; metal salts of organic acids such as sodium acetate and sodium formate; polymers such as polyethylene glycol and polyvinyl pyrrolidone; polymer electrolytes such as sodium polystyrene sulfonate and polyvinylbenzyl trimethyl ammonium chloride; and ionic surfactants such as sodium dioctyl sulfosuccinate and sodium alkylmethyl taurate. The additive may act as a swelling agent for the porous structure.

The porous membrane is preferably a membrane formed from one composition as a single layer and preferably does not have a laminate structure of a plurality of layers.

The method for producing a porous membrane is not particularly limited as long as the porous membrane having the above-described structure can be formed, and any general method for forming a polymer membrane can be used. Examples of the method for forming a polymer membrane include a stretching method and a flow casting method.

For example, in the flow casting method, a porous membrane having the above-described structure can be produced by adjusting the kind and the amount of the solvent used in the membrane-forming stock solution, and the drying method after flow casting.

The production of the porous membrane by the flow casting method can be carried out, for example, by a method including the following (1) to (4) in the following order.
(1) A membrane-forming stock solution containing a polymer, an additive as necessary, and a solvent as necessary is flow-cast on a support in a dissolved state.
(2) A temperature-controlled wet air is applied to the surface of the flow-cast liquid membrane.
(3) The membrane obtained after applying the temperature-controlled wet air is immersed in the solidification liquid.
(4) The support is peeled off as necessary.

The temperature of the temperature-controlled wet air may be 4°C to 60°C and preferably 10°C to 40°C. The relative humidity of the temperature-controlled wet air may be 30% to 70% and preferably 40% to 50%. The absolute humidity of the temperature-controlled wet air is preferably 1.2 to 605 g/kg air, and more preferably 2.4 to 300 g/kg air. The temperature-controlled wet air may be applied at a wind speed of 0.1 m/sec to 10 m/sec for 0.1 seconds to 30 seconds and preferably 1 second to 10 seconds.

The average pore diameter and the position of the dense portion can be controlled by the concentration of water contained in the temperature-controlled wet air and the time during the temperature-controlled wet air is applied. The average pore diameter of the dense portion can also be controlled by the amount of water contained in the membrane-forming stock solution.

In a case where the temperature-controlled wet air is applied to the surface of the liquid membrane as described above, the vaporization of the solvent can be controlled and coacervation can be caused to occur from the surface of the liquid membrane toward the inside. In this state, in a case where the membrane is immersed in a solidification liquid containing a solvent in which solubility of a polymer is low but which is compatible with the solvent of the polymer, the coacervation phase is fixed as micropores and pores other than the micropores can be formed.

The temperature of the solidification liquid may be -10°C to 80°C in the process of immersing in the solidification liquid. In a case where the temperature is changed in this range, the time taken from the formation of the coacervation phase, from the dense portion to the support surface side, to the solidification is adjusted, and the size of the pore diameter up to the surface on the support surface side can be controlled. In a case where the temperature of the solidification liquid is raised, the formation of the coacervation phase is accelerated and the time taken for reaching solidification is lengthened, and thus the pore diameter toward the support surface side easily becomes large. On the other hand, in a case where the temperature of the solidification liquid is lowered, the formation of the coacervation phase is decelerated and the time taken for reaching solidification is shortened, and thus the pore diameter toward the support surface side does not easily become large.

As the support, a plastic film or a glass plate may be used. Examples of the material of the plastic membrane include polyester such as polyethylene terephthalate (PET), polycarbonate, an acrylic resin, an epoxy resin, polyurethane, polyamide, polyolefin, a cellulose derivative, and silicone. As the support, a glass plate or PET is preferable, and PET is more preferable.

The membrane-forming stock solution may contain a solvent. As the solvent, a solvent in which a solubility of a polymer to be used is high (hereinafter, may be referred to as "good solvent") may be used depending on the polymer to be used. The good solvent is preferably one that is quickly replaced with a solidification liquid in a case where the membrane is immersed in the solidification liquid. Examples of the solvent include N-methyl-2-pyrrolidone, dioxane, tetrahydrofuran, dimethylformamide, dimethylacetamide, and a mixed solvent thereof in a case where the polymer is polysulfone or the like; dioxane, N-methyl-2-pyrrolidone, dimethylformamide, dimethylacetamide, dimethylsulfoxide, and a mixed solvent thereof in a case where the polymer is polyacrylonitrile or the like; dimethylformamide, dimethylacetamide, or a mixed solvent thereof in a case where the polymer is polyamide or the like; and acetone, dioxane, tetrahydrofuran, N-methyl-2-pyrrolidone, and a mixed solvent thereof in a case where the polymer is cellulose acetate or the like.

As the membrane-forming stock solution, in addition to the good solvent, it is preferable to use a solvent in which solubility of a polymer is low but which is compatible with the solvent of the polymer (hereinafter, may be referred to as a "non-solvent"). Examples of the non-solvent include water, cellosolves, methanol, ethanol, propanol, acetone, tetrahydrofuran, polyethylene glycol, and glycerin. Among these, water is preferably used.

The concentration of the polymer as a membrane-forming stock solution may be 5% by mass or more and 35% by mass or less and preferably 10% by mass or more and 30% by mass or less. In a case of being 35% by mass or less, sufficient permeability (for example, water permeability) can be imparted to the obtained porous membrane, and in a case of being 5% by mass or more, it is guaranteed to form a porous membrane that selectively permeates a substance. The amount of the additive added is not particularly limited as long as the uniformity of the membrane-forming stock solution is not lost by the addition; however, it is generally 0.5% by mass or more and 10% by mass or less with respect to the solvent. In a case where the membrane-forming stock solution contains a non-solvent and a good solvent, the proportion of the non-solvent to the good solvent is not particularly limited as long as the mixed solution can maintain a uniform state; however, it is preferably 1.0% by mass to 50% by mass, more preferably, 2.0% by mass to 30% by mass, and still more preferably 3.0% by mass to 10% by mass.

As the solidification liquid, it is preferable to use a solvent in which a solubility of a polymer is low. Examples of such a solvent include water, alcohols such as methanol, ethanol, and butanol, glycols such as ethylene glycol and diethylene glycol, aliphatic hydrocarbons such as ether, n-hexane, and n-heptane, and glycerols such as glycerin. Examples of the preferred solidification liquid include water, alcohols, and a mixture of two or more thereof. Among these, water is preferably used.

After immersion in the solidification liquid, it is also preferable to carry out washing with a solvent different from the solidification liquid used. Washing can be carried out by immersion in a solvent. The washing solvent is preferably diethylene glycol. In a case where diethylene glycol is used as a washing solvent and one or both of the temperature of the diethylene glycol in which the membrane is immersed and the immersion time are adjusted, the distribution of N element in the porous membrane can be adjusted. In particular, in a case where polyvinyl pyrrolidone is used as the membrane-forming stock solution of the porous membrane, the remaining amount of polyvinyl pyrrolidone in the membrane can be controlled. After washing with diethylene glycol, washing with water may be carried out.

The membrane-forming stock solution for the porous membrane is preferably a membrane-forming stock solution obtained by dissolving polysulfone and polyvinyl pyrrolidone in N-methyl-2-pyrrolidone and adding water thereto.

Regarding the method for producing a porous membrane, JP1992-349927A (JP-H04-349927A), JP1992-068966B (JP-H04-068966B), JP1992-351645A (JP-H04-351645A), JP2010-235808A, and the like can be referred to.

### <Second sac-shaped structure>

The second sac-shaped structure has an average long diameter larger than the average pore diameter of the pores of the first sac-shaped structure. At least a part of the plurality of second sac-shaped structures contain cells.

The average long diameter can be measured by taking an image of a culture dish containing 50 or more of the prepared second sac-shaped structures with a BZ-X all-in-one fluorescence microscope (KEYENCE Corporation) or Cell3iMager (Shimadzu Corporation) and analyzing the taken image with ImageJ.

The material of the second sac-shaped structure is preferably a polysaccharide hydrogel, a polypeptide, a polylactic acid, a polyglycolic acid, a copolymer of lactic acid and glycolic acid, hyaluronic acid, glycosaminoglycan, proteoglycan, chondroitin, cellulose, agarose, carboxymethyl cellulose, chitin, chitosan, alginic acid, sodium alginate, calcium alginate, potassium alginate, barium alginate, alginate, a layered matrix of polylysine, a layered matrix of polyornithine, a photopolymerized polymer, polyacrylate, hydroxyethyl methacrylate, methyl methacrylate, silicone (a silicone capsule and a silicone nanocapsule), a polyether sulfone membrane, an acrylonitrile-vinyl chloride copolymer, or a combination thereof. Among the above, a material having biocompatibility is preferable, and sodium alginate is most preferable.

The membrane constituting the second sac-shaped structure is preferably a selectively permeable membrane that is capable of blocking substances having a molecular weight of 500 kDa or more, more preferably 100 kDa or more, still more preferably 80 kDa or more, and particularly preferably 50 kDa or more. The membrane of the sodium alginate microcapsule that is used in Examples which will be described later can block substances of 500 kDa or more for 24 hours or more and can block substances of 150 kDa or more for 2 hours or more.

In the present invention, all of the plurality of second sac-shaped structures may contain cells, or at least a part of the plurality of second sac-shaped structures may do not contain cells.

The proportion of the second sac-shaped structure that does not contain cells is preferably 50% or less, more preferably 40% or less, and still more preferably 30% or less.

The cells contained in the second sac-shaped structure are not particularly limited; however, cells producing a useful substance are preferable. Examples of the useful substance include a hormone (insulin or the like), a cytokine (interferon, interleukin, tumor necrosis factor, colony stimulating factor, growth factor, or the like), an enzyme (a lysosomal storage disease-related enzyme or the like), a neurotransmitter (dopamine or the like), an antibody, an antigen, and another physiologically active substance (a protein, a peptide, or the like.); however, the examples are not limited thereto. The cells that produce a useful substance may be non-transformed cells such as pancreatic cells (β cells, and the like), skin cells, cartilage cells, liver cells, and renal cells, or may be transformed cells into which a gene encoding a useful substance or a gene involved in the biosynthesis of a useful substance is introduced. Further, the cells may be cells derived from a living body or cells derived from an embryonic stem cell or an induced pluripotent stem cell.

Among the above, the cells are preferably insulin-secreting cells.

The cells may be a cell aggregate. The cell aggregate is a cell mass in which a plurality of cells are aggregated.

As one example of the cell aggregate, a pancreatic islet can be used.

In a case where the second sac-shaped structure contains cell aggregates, the average number of cell aggregates contained in one second sac-shaped structure is preferably 1.0 or more and 5.0 or less, more preferably 1.0 or more and 4.0 or less, still more preferably 1.0 or more and 3.0 or less, and particularly preferably 1.0 or more and 2.0 or less. The average is calculated without including the number of second sac-shaped structures that do not contain cell aggregates.

The second sac-shaped structure may contain mesenchymal stem cells in addition to insulin-secreting cells. The mesenchymal stem cells may be encapsulated preferably as a cell aggregate and more preferably as a cell structure containing mesenchymal stem cells. The cell structure containing mesenchymal stem cells will be described later in the present specification.

Between the first sac-shaped structure and the second sac-shaped structure (that is, the space inside the first sac-shaped structure and outside the second sac-shaped structure), mesenchymal stem cells may be contained. The mesenchymal stem cells may be encapsulated as a cell aggregate or as a cell structure containing mesenchymal stem cells. The cell structure containing mesenchymal stem cells will be described later in the present specification.

The second sac-shaped structure containing cells can be produced by suspending the cells in a polymer solution. In a case where a pancreatic islet is used as cells, a sac-shaped structure containing the pancreatic islet can be produced by suspending the pancreatic islet in a polymer solution. According to the present invention, a method for producing a sac-shaped structure containing a pancreatic islet, which includes suspending a pancreatic islet in a polymer solution of 0.5% to 4% by mass and in which a volume of the polymer solution per pancreatic islet is 0.02 to 2.0 µL, is provided. The concentration of the polymer solution at the time of suspending the pancreatic islet may be 0.5% to 4% by mass, preferably 1.0% to 3% by mass, and more preferably 1.5% to 2.5% by mass. The volume of the polymer solution per pancreatic islet is 0.01 to 2.0 µL, preferably 0.05 to 0.5 µL, and more preferably 0.07 to 0.2 µL.

### <Cell structure>

The cell transplantation kit according to the embodiment of the present invention further contains a cell structure including a polymer block having biocompatibility and cells, in which a plurality of the polymer blocks are arranged in a gap between the plurality of cells.

In a case where a plurality of polymer blocks, using polymer blocks having biocompatibility and cells, are three dimensionally arranged in the mosaic pattern in the cell gap, the polymer block having biocompatibility and the cells are three dimensionally arranged in the mosaic pattern, and thus a cell three-dimensional structure in which the cells are uniformly present in the structure is formed.

In the cell structure, the plurality of polymer blocks are arranged in the gaps between a plurality of cells. However, here, the "cell gap" needs not to be a space closed by the constituent cells and may be sandwiched between cells. All cells need not have gaps, and there may be a place where cells are in contact with each other. The distance of the cell gap interposed by the polymer block, that is, the gap distance between a certain cell and a cell that is present at the shortest distance from the cell is not particularly limited; however, the distance is preferably a distance of the size of the polymer block, and the suitable distance is also a distance in the range of the suitable size of the polymer block.

Further, although the polymer block has a configuration sandwiched between cells, it is not necessary that cells are present between all the polymer blocks, and there may be a place where the polymer blocks are in contact with each other. The distance between the polymer blocks interposed by the cell, that is, the distance between a certain polymer block and a polymer block that is present at the shortest distance from the block is not particularly limited; however, the distance is preferably a distance of a size of a cell mass formed by aggregation of one to several cells to be used, and for example, it is 10 µm or more and 1,000 µm or less, preferably 10 µm or more and 100 µm or less, and more preferably 10 µm or more and 50 µm or less.

The polymer constituting the biocompatible polymer block is not particularly limited as long as it has the biocompatibility to the living body, and it is not particularly limited whether or not the polymer is degraded in the living body; however, the polymer is preferably composed of a biodegradable material. Specifically, the biodegradable material is at least one material selected from the group consisting of a polypeptide, a polylactic acid, a polyglycolic acid, a copolymer of lactic acid and glycolic acid (PLGA), hyaluronic acid, glycosaminoglycan, proteoglycan, chondroitin, cellulose, agarose, carboxymethyl cellulose, chitin, and chitosan. Among the above, a polypeptide is particularly preferable. The kind of the polypeptide is not particularly limited as long as the polypeptide has biocompatibility; however, for example, gelatin, collagen, elastin, fibronectin, ProNectin, laminin, tenascin, fibrin, fibroin, entactin, thrombospondin, or RetroNectin is preferable, and gelatin, collagen, or atelocollagen is most preferable. As the gelatin to be used in the present invention, a natural gelatin or a recombinant gelatin is preferable. A recombinant gelatin is more preferable.

Details and preferred embodiments of the cell structure are described in WO2011/108517A, JP2014-012114A, WO2014/133581A, JP2015-134193A, and WO2015/190430A.

The kind of cell in the cell structure is not particularly limited; however, the cell is preferably a somatic stem cell.

Examples of the somatic stem cell capable of being used include a mesenchymal stem cell (MSC), a hematopoietic stem cell, an amnion cell, an umbilical cord blood cell, and a bone marrow-derived cell (for example, a bone marrow-derived MCS), a myocardial stem cell, an adipose-derived stem cell, and a neural stem cells. Among the above, a mesenchymal stem cell is preferable, an adipose-derived mesenchymal stem cell or a bone marrow-derived mesenchymal stem cell is more preferable, and an adipose-derived mesenchymal stem cell is still more preferable. The mesenchymal stem cell refers to a somatic stem cell present in the mesenchymal tissue and has an ability to differentiate into a cell belonging to the mesenchymal tissue. The mesenchymal tissue refers to tissue such as bone, cartilage, fat, blood, bone marrow, skeletal muscle, dermis, ligament, tendons, or heart.

### <Plate-shaped cell non-adhesive material>

The cell transplantation kit according to the embodiment of the present invention may further include a plate-shaped cell non-adhesive material. The plate-shaped cell non-adhesive material is a material that is used as a material for maintaining the space in the inside of the first sac-shaped structure in the process of forming a coat around the first sac-shaped structure in the living body. Silicone is preferred as the cell non-adhesive material.

The shape of the cell non-adhesive material may be a plate shape, and specific examples thereof include a rectangular parallelepiped and a cylinder; however, the shape is not particularly limited. The size of the rectangular parallelepiped can be about 0.5 cm to 3 cm in height, 0.5 cm to 3 cm in width, and 0.5 mm to 5 mm in thickness; however, the size is not particularly limited.

### <Application>

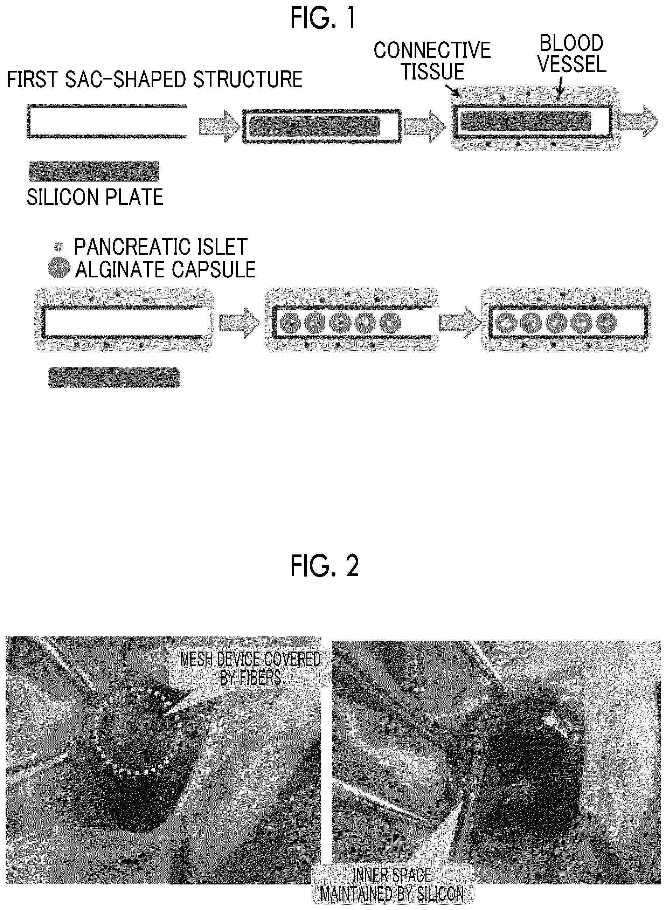

A schematic view illustrating an operation in a case where a pancreatic islet is transplanted using one example of a cell transplantation kit according to the present invention is shown in Fig. 1.

As the application of the cell transplantation kit of the present invention, the first sac-shaped structure is introduced into a living body, a coat derived from a recipient is formed around the first sac-shaped structure introduced into the living body, and then the plurality of second sac-shaped structures are incorporated into the first sac-shaped structure.

In one example, the first sac-shaped structure containing a cell non-adhesive material can be sewn to an abdominal cavity side of a peritoneum to introduce the first sac-shaped structure into the living body.

In the process of forming a coat derived from a recipient around the first sac-shaped structure, at least one of a cytokine, a mesenchymal stem cell, an extracellular matrix, or collagen may be incorporated into the first sac-shaped structure.

In the process of forming a coat derived from a recipient around the first sac-shaped structure, the first sac-shaped structure may be left at the transplantation site; however, the first sac-shaped structure is preferably fixed to muscle or skin by suturing.

Examples of the site for transplanting the first sac-shaped structure may include the abdominal cavity, a subcutaneous site, peritoneum of a recipient, which are not particularly limited.

The recipient means a living body that is subjected to transplantation. The recipient is preferably a mammal and more preferably a human.

In the present invention, the pancreatic islet and the cell from the donor may be allogeneic or heterologous. Preferably, the pancreatic islet is of heterologous origin and the cells are of allogeneic origin. In particular, in a case where a mesenchymal stem cell is used as the cell, a mesenchymal stem cell of allogeneic origin is preferable.

The cell transplantation kit according to the embodiment of the present invention may be used for only one transplantation or may be used for twice or more transplantations as necessary.

The cell transplantation kit according to the embodiment of the present invention can be used for the medical treatment of the disease, requiring cell transplantation. Examples of the medical treatment of diseases, requiring cell transplantation, include a diabetes medical treatment using pancreatic cells (β cells) or insulin-producing cells, a lysosomal storage disease-related medical treatment using cells that secrete a lysosomal storage disease-related enzyme, and a medical treatment of Parkinson's disease using dopamine-producing cells; however, the examples are not limited thereto.

### <Various applications>

According to the present invention, a cell transplantation method including a process of introducing a first sac-shaped structure consisting of a membrane having pores having an average pore diameter of 0.2 mm or less into a living body, forming a coat derived from a recipient around the first sac-shaped structure introduced into the living body, and then incorporating a plurality of second sac-shaped structures into the first sac-shaped structure, where the second sac-shaped structures have an average long diameter larger than the average pore diameter of the pores of the first sac-shaped structure and at least a part of the plurality of second sac-shaped structures contain cell, is provided.

The cell transplantation kit of the present invention, including the combination of a first sac-shaped structure consisting of a membrane having pores of an average pore diameter of 0.2 mm or less, and a plurality of second sac-shaped structures having an average long diameter larger than the average pore diameter of the pores of the first sac-shaped structure, where at least a part of the plurality of second sac-shaped structures contain cells, can be used in the medical treatment of a disease requiring cell transplantation.

The combination of a first sac-shaped structure, consisting of a membrane having pores of an average pore diameter of 0.2 mm or less, and a plurality of second sac-shaped structures having an average long diameter larger than the average pore diameter of the pores of the first sac-shaped structure, where at least a part of the plurality of second sac-shaped structures contain cells, can be used for manufacturing the cell transplantation therapy kit of the present invention.

The present invention will be further specifically described with reference to Examples, but the present invention is not limited by Examples.

### Examples

<Example 1> Evaluation of performance of heterologous pancreatic islet transplantation using first sac-shaped structure made of nylon.

### (1) Preparation of first sac-shaped structure

Two nylon meshes (Corning Inc., Falcon (registered trade mark) 40 µm cell strainer) having an average pore diameter of 40 µm were cut out in a circle shape of a diameter of 2 cm and overlapped, and three sides thereof were sealed by heating using a tea sac sealer (T-230K) manufactured by FUJI IMPULSE Co., Ltd. so that space was created in the inside. Then, silicone having a height of 1 cm, a width of 1 cm, and a thickness of 1 mm was inserted from the remaining one unsealed side, and then the side into which the silicone was inserted was sealed with the sealer above to form a first sac-shaped structure.

### (2) Formation of coat around first sac-shaped structure

The first sac-shaped structure prepared in (1) above was transplanted into the abdominal cavity of a 6-week-old Balb/c mouse. At the time of transplantation, two end portions of the first sac-shaped structure were sewn to the abdominal wall to fix the position, and a coat derived from a recipient was formed around the first sac-shaped structure.

### (3) Preparation of diabetic mice

Three weeks after transplanting the first sac-shaped structure in (2) above, streptozotocin (manufactured by FUJIFILM Wako Pure Chemical Corporation) was intraperitoneally administered at a dose of 250 mg/kg per mouse. One week after administration of streptozotocin, a mouse having a blood glucose level of 300 mg/dL was used as a diabetic-developed mouse as a recipient of cell transplantation.

### (4) Isolation of rat pancreatic islet

A Wistar rat was prepared, and 1 mg/mL Collagenase type V (manufactured by Sigma-Aldrich Co., LLC) was injected through the pancreatic duct to swell pancreas and removed the swollen pancreas. The removed pancreas was digested at 37°C, and then pancreatic islet was purified by density gradient centrifugation at 900 g using 10 mL of Lymphoprep (manufactured by Cosmo Bio Co., Ltd.) and 10 mL of HBSS (FUJIFILM Wako Pure Chemical Corporation) for 20 minutes.

### (5) Preparation of sodium alginate microencapsulated pancreatic islet

Pancreatic islet isolated from Wistar rats was suspended in a 1.8% sodium alginate solution (manufactured by BUCHI Labortechnik AG) so that the rate of 0.06 to 0.1 µL per pancreatic islet was maintained. The pancreatic islet was uniformly dispersed by repeatedly moving the entire suspension between two syringes. Using Encapsulator B-395 Pro manufactured by BUCHI Labortechnik AG, the suspension was dropwise added to 100 mM calcium chloride under stirring, whereby a sodium alginate microencapsulated pancreatic islet was obtained. The alginate capsules were coated with 0.05% by mass of poly-L-Lysine for 2 minutes.

### (6) Evaluation of sodium alginate-encapsulated pancreatic islet

10% of the prepared capsules were extracted, images were taken with Cell3iMager (Shimadzu Corporation), and image analysis was carried out with ImageJ. The proportion of the empty capsules was calculated by counting all the capsules and the empty capsules in the image plane. The average number of pancreatic islets in the capsule was calculated by measuring the total number of pancreatic islets and the number of capsules containing the pancreatic islets in the image plane. Among the capsules prepared, 26.4% were empty capsules that did not contain pancreatic islets. The average number of pancreatic islets per capsule was 1.70.

### (7) Preparation of cell structure

Mouse fat-derived mesenchymal stem cells (mADSC) were suspended in D-MEM medium (Dulbecco's modified Eagle's medium) containing 10% fetal bovine serum (FBS), and a biocompatible polymer block (53 to 106 µm) (the biocompatible polymer block described in Example 2 of JP2015-134193A) was added thereto. In a final state where mADSC (1.2 × 10⁶ cells) and the biocompatible polymer block (0.25 mg) were suspended in 4 ml of the medium, the suspension was seeded in an EZSPHERE (registered trade mark) dish Type 903 (spheroid well diameter: 800 µm, spheroid well depth: 300 µm, spheroid well number: about 1,200 wells, the bottom surface is a culture surface having a recessed portion, and has an outer side wall portion erected on the peripheral edge of the culture surface, manufactured by AGC TECHNO GLASS Co., Ltd.), which is a cell non-adhesive 35 mm dish. The dish was allowed to stand in a CO₂ incubator at 37°C for 48 hours to obtain about 1,200 cell structures which were uniform.

### (8) Transplantation of sodium alginate microencapsulated pancreatic islet

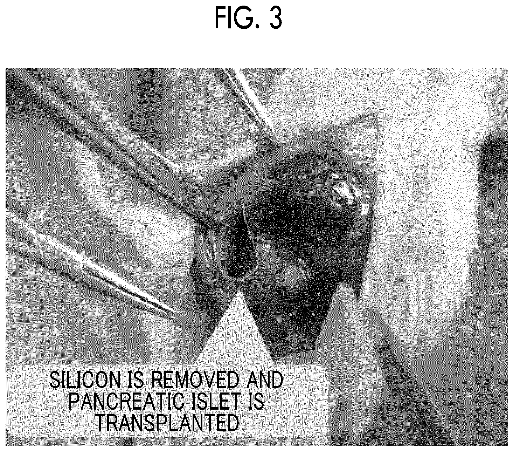

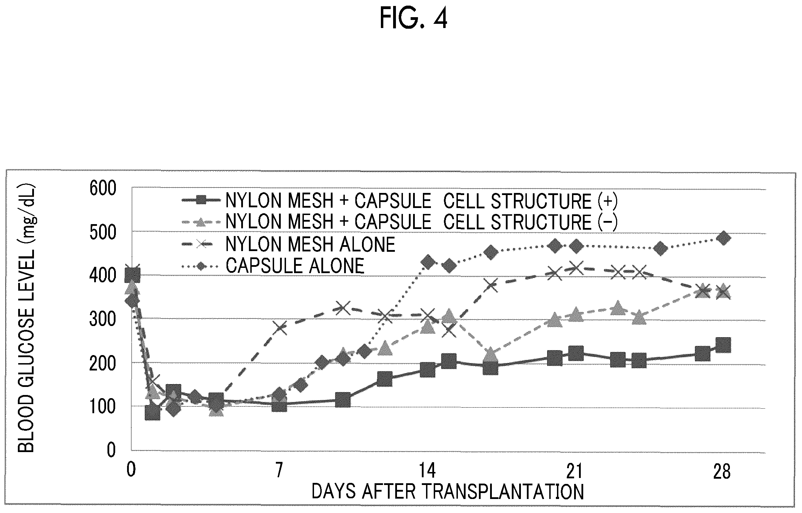

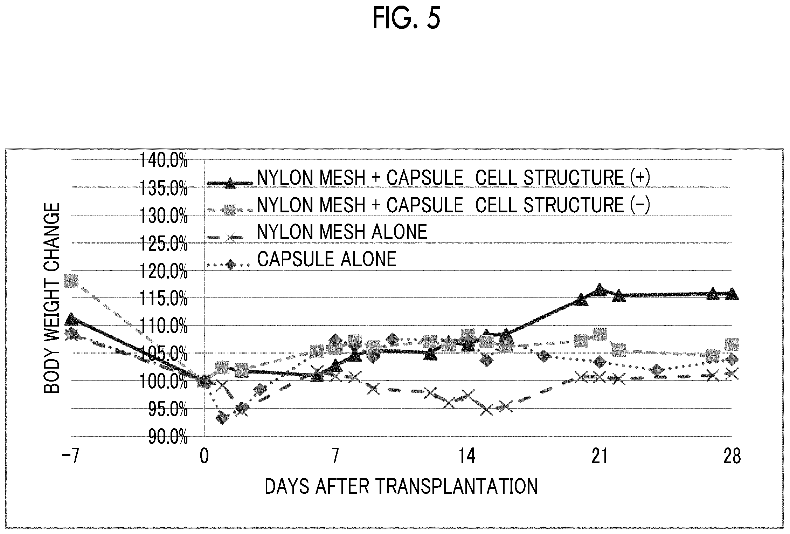

At the moment of four weeks after the first sac-shaped structure was transplanted into the diabetic mouse prepared in (3) above (Fig. 2), the diabetic mouse was opened, one side of the first sac-shaped structure covered by the coat was cut with a scissors, and the silicone inside was removed. The sodium alginate microencapsulated pancreatic islet obtained in (5) above was transplanted to the space created after removing the silicone, and the cell transplantation was completed by sealing the first sac-shaped structure (Fig.3). After the completion of the cell transplantation, the blood glucose level and the body weight of the mouse were observed over time. Changes in blood glucose level and body weight are shown in Fig. 4 and Fig. 5.

### (9) Sodium alginate microencapsulated pancreatic islet and cell structure transplantation

At the moment of four weeks after the first sac-shaped structure was transplanted into the diabetic mouse prepared in (3) above (Fig. 2), the diabetic mouse was opened, one side of the first sac-shaped structure covered by the coat was cut with a scissors, and the silicone inside was removed. The sodium alginate microencapsulated pancreatic islet obtained in (5) above and the cell structure obtained in (7) above were mixed and transplanted to the space created after removing the silicone, and the cell transplantation was completed by sealing the first sac-shaped structure. After the completion of the cell transplantation, the blood glucose level and the body weight of the mouse were observed over time. Changes in blood glucose level and body weight are shown in Fig. 4 and Fig. 5.

### <Comparative Example 1>

As Comparative Example 1, the experiment was carried out by carrying out the same operation as in (8) of Example 1, except that sodium alginate microencapsulation was not carried out.

As a result of the experiment, the average blood glucose level was 280.8 mg/dL (SD: 182.7) at 7 days, 310.6 mg/dL (SD: 144.7) at 14 days, 421 mg/dL (SD: 145.1) at 21 days, and 366 mg/dL (SD: 152.5) at 28 days after transplantation, and it was found that the blood glucose level cannot be controlled at 7 days. The body weight was 101.4% (SD: 1.9) at 28 days, assuming that the body weight on the day of transplantation was 100%.

On the other hand, in the mouse subjected to the transplantation with the first sac-shaped structure and the sodium alginate microencapsulated pancreatic islet, which was carried out in (8), the average blood glucose level was 130 mg/dL (SD: 80.6) at 7 days, 286 mg/dL (SD: 121.8) at 14 days, 314.8 mg/dL (SD: 138.1) at 21 days, and 371 mg/dL (SD: 132.2) at 28 days after transplantation, and it has been shown that the configuration of the first sac-shaped structure and sodium alginate microencapsulation is useful for exhibiting a medical treatment effect since blood glucose level control was confirmed for more than 7 days. The body weight was 106.6% (SD: 2.9) at 28 days, assuming that the body weight on the day of transplantation was 100%, and it was found that the body weight was increased as compared with Comparative Example 1. Further, in the case of transplanting the cell structure carried out in (9), the average blood glucose level was 106.2 mg/dL (SD: 33.0) at 7 days, 186.2 mg/dL (SD: 103.4) at 14 days, 226.4 mg/dL (SD: 105.7) at 21 days, and 245.8 mg/dL (SD: 86.8) at 28 days after transplantation. Even 28 days after the transplantation, the average blood glucose level was below 250 mg/dL, indicating that more strong action of normalization of the blood glucose level is exhibited. The body weight was 115.8% (SD: 2.92) at 28 days, assuming that the body weight on the day of transplantation was 100%, and it was found that high function of glucose metabolism is exhibited as compared with Comparative Example 1 and (8) of Example.

### <Comparative Example 2>

As Comparative Example 2, the experiment was carried out by carrying out the same operation as in (8) of Example 1, except that the first sac-shaped structure was not used.

As a result of the experiment, in the case of transplantation in which the first sac-shaped structure was not used (Comparative Example 2), the average blood glucose level was 103.6 mg/dL (SD: 12.9) at 7 days, 432 mg/dL (SD: 48.1) at 14 days, 470.2 mg/dL at 21 days (SD: 32.9), and 489 mg/dL (SD: 64.8) at 28 days after transplantation. The body weight was 103.9% (SD: 8.2) at 28 days, assuming that the body weight on the day of transplantation was 100%.

In addition, a state in which the blood glucose level was 250 mg/mL or less in two consecutive measurements was determined to be completely cured, and the rate of the complete cure was defined as the complete cure rate. The complete cure rates were calculated in Comparative Example 1, Comparative Example 2, and (8) and (9) of Example, and the results are shown in Table 1. In a case of viewing the results in terms of the complete cure rate, it has been found that the configuration including the cell structure exhibits a remarkably high complete cure rate.

**[Table 1]**

| N = 5 | Comparative Example 2 Capsule alone | Comparative Example 1 Nylon mesh alone | (8) of Example Nylon mesh + capsule Cell structure (-) | (9) of Example Nylon mesh + capsule Cell structure (+) |
|---|---|---|---|---|
| Complete cure rate | 0% | 20% | 20% | 80% |

### (Observation of tissue specimen)

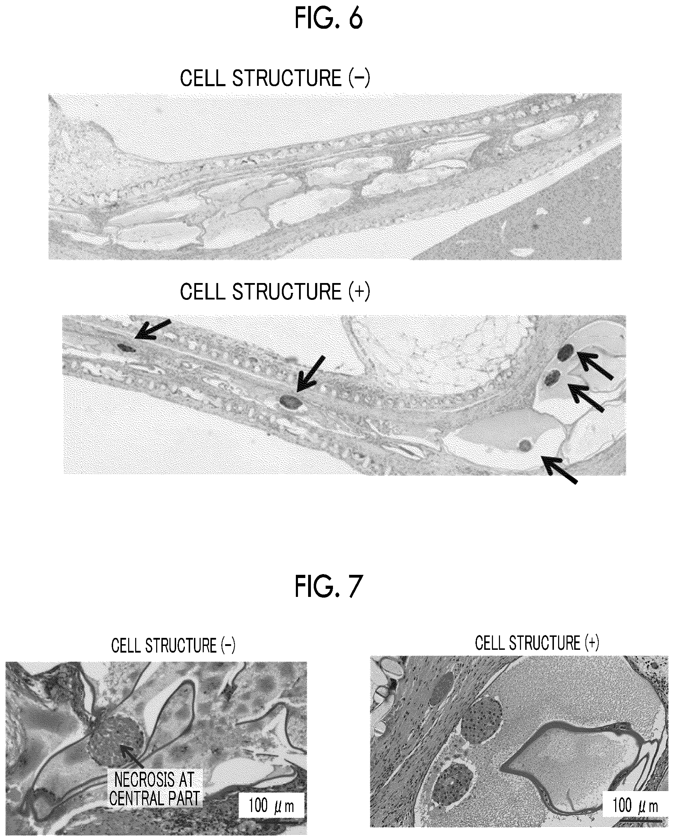

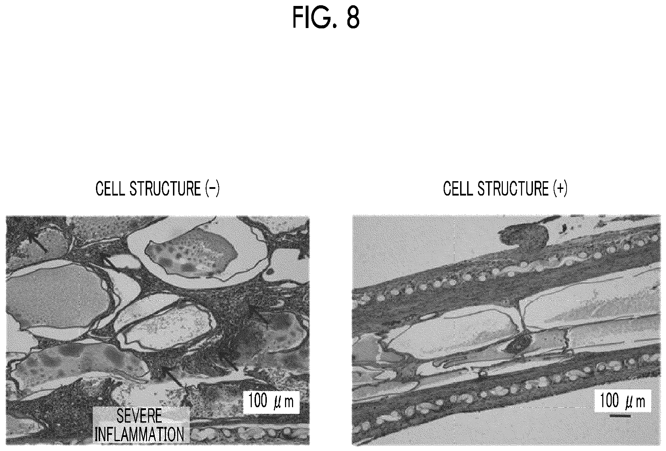

A mouse 4 weeks after the transplantation of the pancreatic islet was opened under anesthesia, and the sutured muscle and the tissue covering the nylon mesh device were removed together. The removed specimen was fixed with a 10% formalin neutral buffer (FUJIFILM Wako Pure Chemical Corporation) and then embedded in paraffin to prepare a slice of a tissue specimen. The prepared slice was stained with hematoxylin and eosin (HE) and immunostained with an anti-insulin antibody (Abcam plc). As a result of observing the tissue specimen, it has been confirmed that a slight insulin-positive region was confirmed in the cell structure (-) group. On the other hand, many insulin-positive regions were observed in the cell structure (+) group (Fig. 6). Regarding the pancreatic islet survival, the pancreatic islet of which the central part or the entire part were dead by necrosis were observed in the cell structure (-) group. On the other hand, in the cell structure (+) group, many viable pancreatic islets in which nuclei were stained to the central part thereof were observed (Fig. 7). Regarding the inflammatory response, many inflammatory immune cells were present around the capsule in the cell structure (-) group, but few inflammatory cells were found around the capsule in the cell structure (+) group Fig. 8).

The results of tissue specimen observation are shown in Table 2. Regarding the number of insulin-positive regions, the number of insulin-positive regions detected per specimen is indicated by the following notations, 0: C, 1 to 4: B, and 5 or more: A. Regarding the pancreatic islet survival, the number of nuclear-stained pancreatic islets per specimen is indicated by the following notations, 0: C, 1 to 4: B, and 5 or more: A. Regarding the inflammation around the capsule, the proportion of the area of the inflammatory cell region in the tissue specimen of the specimen is indicated by the following notations, 0% to less than 20%: A, 20% to less than 50%: B, and 50% to 100%: C.

**[Table 2]**

| | Insulin-positive region | Pancreatic islet survival | Inflammation around capsule |
|---|---|---|---|
| Capsule (-) | C | C | - |
| Cell structure (-) | B | B | B |
| Cell structure (+) | A | A | A |

## Claims

1. A cell transplantation kit comprising:
a first sac-shaped structure consisting of a membrane having pores of an average pore diameter of 0.2 mm or less;
a plurality of second sac-shaped structures having an average long diameter larger than the average pore diameter of the pores of the first sac-shaped structure; and
a cell structure including a polymer block having biocompatibility and cells,
wherein
the plurality of second sac-shaped structures are provided for incorporation into the first sac-shaped structure,
at least a part of the plurality of second sac-shaped structures contain cells,
in the cell structure, a plurality of the polymer blocks is arranged in a gap between a plurality of the cells, and
the cell structure is provided inside of the first sac-shaped structure.

2. The cell transplantation kit according to claim 1,
wherein a material of the first sac-shaped structure includes nylon, polysulfone, polytetrafluoroethylene, polyurethane, polyester, vinyl chloride, polycarbonate, acrylic, stainless steel, titanium, silicon, or 2-methacryloyloxyethyl phosphorylcholine.

3. The cell transplantation kit according to claim 1 or 2,
wherein a material of the second sac-shaped structure includes a polysaccharide hydrogel, a polypeptide, a polylactic acid, a polyglycolic acid, a lactic acid/glycolic acid copolymer, hyaluronic acid, glycosaminoglycan, proteoglycan, chondroitin, cellulose, agarose, carboxymethyl cellulose, chitin, chitosan, alginic acid, sodium alginate, calcium alginate, potassium alginate, barium alginate, alginate, a layered matrix of polylysine, a layered matrix of polyornithine, a photopolymerized polymer, polyacrylate, hydroxyethyl methacrylate, methyl methacrylate, silicon, a polyether sulfone membrane, an acrylonitrile-co-vinyl chloride, or a combination thereof.

4. The cell transplantation kit according to any one of claims 1 to 3,
wherein a proportion of the second sac-shaped structure that does not contain cells is 50% or less.

5. The cell transplantation kit according to any one of claims 1 to 4,
wherein the cells contained in the second sac-shaped structure are a cell aggregate.

6. The cell transplantation kit according to claim 5,
wherein the cell aggregate is a pancreatic islet.

7. The cell transplantation kit according to claim 5 or 6,
wherein regarding the second sac-shaped structure containing the cell aggregate, an average number of the cell aggregates contained in one second sac-shaped structure is 1.0 or more and 5.0 or less.

8. The cell transplantation kit according to any one of claims 1 to 7,
wherein the cells contained in the cell structure are mesenchymal stem cells.

9. The cell transplantation kit according to any one of claims 1 to 8, further comprising a plate-shaped cell non-adhesive material.

10. A method for producing the cell transplantation kit according to claim 1, comprising the step of providing the cell transplantation kit of claim 1, wherein the second sac-shaped structure contains a pancreatic islet, the method comprising suspending a pancreatic islet in 1.0% to 3% by mass of a polymer solution,
wherein a volume of the polymer solution per pancreatic islet is 0.05 to 0.5 µL.

## Patentansprüche

1. Zelltransplantationskit, umfassend:
eine erste sackförmige Struktur, bestehend aus einer Membran, die Poren mit einem mittleren Porendurchmesser von 0,2 mm oder kleiner aufweist;
eine Vielzahl von zweiten sackförmigen Strukturen mit einem mittleren Langdurchmesser, der größer ist als der mittlere Porendurchmesser der Poren der ersten sackförmigen Struktur; und
eine Zellstruktur, umfassend einen Polymerblock mit Biokompatibilität und Zellen,
worin die Vielzahl der zweiten sackförmigen Strukturen für die Einarbeitung in die erste sackförmige Struktur vorgesehen sind,
zumindest ein Teil der Vielzahl der zweiten sachförmigen Strukturen Zellen enthält,
in der Zellstruktur eine Vielzahl der Polymerblöcke in einer Lücke zwischen einer Vielzahl von Zellen angeordnet ist, und
die Zellstruktur innerhalb der ersten sachförmigen Struktur vorgesehen ist.

2. Zelltransplantationskit gemäß Anspruch 1,
worin ein Material der ersten sackförmigen Struktur Nylon, Polysulfon, Polytetrafluorethylen, Polyurethan, Polyester, Vinylchlorid, Polycarbonat, Acryl, Edelstahl, Titan, Silicium oder 2-Methacryloyloxyethylphosphorylcholin umfasst.

3. Zelltransplantationskit gemäß Anspruch 1 oder 2,
worin ein Material der zweiten sachförmigen Struktur ein Polysaccharid-hydrogel, ein Polypeptid, eine Polymilchsäure, eine Polyglykolsäure, ein Milchsäure/Glykolsäure-Copolymer, Hyaluronsäure, Glycosaminoglycan, Proteoglycan, Chondroitin, Cellulose, Agarose, Carboxymethylcellulose, Chitin, Chitosan, Alginsäure, Natriumalginat, Calciumalginat, Kaliumalginat, Bariumalginat, Alginat, eine geschichtete Matrix von Polylysin, eine geschichtete Matrix von Polyornithin, ein fotopolymerisiertes Polymer, Polyacrylat, Hydroxyethylmethacrylat, Methylmethacrylat, Silicium, eine Polyethersulfonmembran, ein Acrylonitrilco-vinylchlorid oder eine Kombination hiervon umfasst.

4. Zelltransplantationskit gemäß irgendeinem der Ansprüche 1 bis 3,
worin der Anteil der zweiten sackförmigen Struktur, der keine Zellen enthält, 50 % oder weniger beträgt.

5. Zelltransplantationskit gemäß irgendeinem der Ansprüche 1 bis 4,
worin die in der zweiten sackförmigen Struktur enthaltenen Zellen ein Zellaggregat sind.

6. Zelltransplantationskit gemäß Anspruch 5,
worin das Zellaggregat ein Pankreasinselchen ist.

7. Zelltransplantationskit gemäß Anspruch 5 oder 6,
worin im Hinblick auf die zweite sackförmige Struktur, die das Zellaggregat enthält, die mittlere Anzahl der Zellaggregate, die in einer zweiten sackförmigen Struktur enthalten sind, 1,0 oder mehr und 5,0 oder weniger beträgt.

8. Zelltransplantationskit gemäß irgendeinem der Ansprüche 1 bis 7,
worin die in der Zellstruktur enthaltenen Zellen mesenchymale Stammzellen sind.

9. Zelltransplantationskit gemäß irgendeinem der Ansprüche 1 bis 8, ferner umfassend ein plattenförmiges Zell-Nichtadhäsivmaterial.

10. Verfahren zur Herstellung des Zelltransplantationskits gemäß Anspruch 1, umfassend den Schritt des Bereitstellens des Zelltransplantationskits gemäß Anspruch 1, worin
die zweite sackförmige Struktur ein Pankreasinselchen enthält, das Verfahren das Suspendieren eines Pankreasinselchen in einer 1,0- bis 3-massen-%-igen Polymerlösung umfasst,
worin das Volumen der Polymerlösung je Pankreasinselchen 0,05 bis 0,5 µl beträgt.

## Revendications

1. Un kit de transplantation cellulaire comprenant :
une première structure en forme de sac constituée d'une membrane dont les pores ont un diamètre moyen de 0,2 mm ou moins ;
une pluralité de deuxièmes structures en forme de sac ayant un diamètre moyen long supérieur au diamètre moyen des pores de la première structure en forme de sac ; et une structure cellulaire incluant un bloc de polymère ayant une biocompatibilité et des cellules,
dans lequel
la pluralité de deuxièmes structures en forme de sac est prévue pour être incorporée dans la première structure en forme de sac,
au moins une partie de la pluralité de deuxièmes structures en forme de sac contient des cellules,
dans la structure cellulaire, une pluralité de blocs de polymère est disposée dans un espace entre une pluralité des cellules, et
la structure cellulaire est fournie à l'intérieur de la première structure en forme de sac.

2. Le kit de transplantation cellulaire selon la revendication 1,
dans lequel un matériau de la première structure en forme de sac comporte du nylon, du polysulfone, du polytétrafluoroéthylène, du polyuréthane, du polyester, du chlorure de vinyle, du polycarbonate, de l'acrylique, de l'acier inoxydable, du titane, du silicium ou de la 2-méthacryloyloxyéthylphosphorylcholine.

3. Le kit de transplantation cellulaire selon la revendication 1 ou 2,
dans lequel un matériau de la deuxième structure en forme de sac comprend un hydrogel de polysaccharide, un polypeptide, un acide polylactique, un acide polyglycolique, un copolymère d'acide lactique et d'acide glycolique, de l'acide hyaluronique, un glycosaminoglycane, un protéoglycane, de la chondroïtine, de la cellulose, de l'agarose, de la carboxyméthylcellulose, de la chitine, du chitosane, de l'acide alginique, alginate de sodium, alginate de calcium, alginate de potassium, alginate de baryum, alginate, matrice stratifiée de polylysine, matrice stratifiée de polyornithine, polymère photopolymérisé, polyacrylate, méthacrylate d'hydroxyéthyle, méthacrylate de méthyle, silicium, membrane de polyéther sulfone, chlorure d'acrylonitrile et de vinyle, ou une combinaison de ceux-ci.

4. Le kit de transplantation cellulaire selon l'une quelconque des revendications 1 à 3,
dans lequel la proportion de la deuxième structure en forme de sac qui ne contient pas de cellules est de 50 % ou moins.

5. Le kit de transplantation cellulaire selon l'une quelconque des revendications 1 à 4,
dans lequel les cellules contenues dans la deuxième structure en forme de sac sont un agrégat de cellules.

6. Le kit de transplantation cellulaire selon la revendication 5,
dans lequel l'agrégat cellulaire est un îlot pancréatique.

7. Le kit de transplantation cellulaire selon la revendication 5 ou 6,
dans lequel, en ce qui concerne la deuxième structure en forme de sac contenant l'agrégat cellulaire, le nombre moyen d'agrégats cellulaires contenus dans une deuxième structure en forme de sac est de 1,0 ou plus et de 5,0 ou moins.

8. Le kit de transplantation cellulaire selon l'une quelconque des revendications 1 à 7,
dans lequel les cellules contenues dans la structure cellulaire sont des cellules souches mésenchymateuses.

9. Le kit de transplantation cellulaire selon l'une quelconque des revendications 1 à 8, comprenant en outre un matériau cellulaire non adhésif en forme de plaque.

10. Une méthode de fabrication du kit de transplantation cellulaire selon la revendication 1 comprenant l'étape de fourniture du kit de transplantation cellulaire de la revendication 1, dans laquelle la deuxième structure en forme de sac contient un îlot pancréatique, la méthode comprenant la mise en suspension d'un îlot pancréatique dans 1,0 % à 3 % en masse d'une solution de polymère, dans lequel un volume de la solution de polymère par îlot pancréatique est de 0,05 à 0,5 µL.
